# EUROPÄISCHE PATENTSCHRIFT

(11) **EP 1 283 855 B1**
(45) Veröffentlichungstag und Bekanntmachung des Hinweises auf die Patenterteilung: **02.11.2005**
(21) Anmeldenummer: 01943135.2
(22) Anmeldetag: 22.05.2001
(51) Int. Cl.: C09B 23/02, C09B 23/01, G01N 33/533, G01N 33/58, C12Q 1/68

(54) **STABILE NIR-MARKER-FARBSTOFFE AUF DER BASIS VON BENZOPYRYLIUM-POLYMETHINEN**
STABLE NEAR-INFRARED (NIR) MARKER DYES BASED ON BENZOPYRYLIUM-POLYMETHINES
COLORANTS MARQUEURS DU DOMAINE DU PROCHE INFRAROUGE STABLES A BASE DE POLYMETHINES DE BENZOPYRYLIUM

(30) Priorität: 23.05.2000 DE 10025820; 23.05.2000 DE 20022277 U
(43) Veröffentlichungstag der Anmeldung: 19.02.2003
(73) Patentinhaber: Dyomics GmbH, 07745 Jena (DE)
(72) Erfinder: CZERNEY, Peter, 99425 Weimar (DE); FRANK, Wilhelm, 07743 Jena (DE); LEHMANN, Frank, 07749 Jena (DE); SCHWEDER, Bernd, 07743 Jena (DE); WENZEL, Matthias, 07749 Jena (DE)
(74) Vertreter: Oehmke, Volker
(86) Internationale Anmeldenummer: PCT/DE2001/001946
(87) Internationale Veröffentlichungsnummer: WO 2001/090253

(56) Entgegenhaltungen:
- DE-A- 4 341 618
- US-A- 3 567 438
- GADJEV N ET AL: "MONOMETHINE CYANINE DYES CONTAINING BENZ[c,d]INDOLE AND PYRYLIUM END-GROUPS" DYES AND PIGMENTS, ELSEVIER APPLIED SCIENCE PUBLISHERS. BARKING, GB, Bd. 24, Nr. 2, 1994, Seiten 93-98, XP000434501 ISSN: 0143-7208
- DATABASE WPI Section Ch, Week 198829 Derwent Publications Ltd., London, GB; Class E23, AN 1988-202526 XP002181113 & JP 63 141068 A (FUJI PHOTO FILM CO LTD), 13. Juni 1988 (1988-06-13)
- DATABASE WPI Section Ch, Week 198829 Derwent Publications Ltd., London, GB; Class E23, AN 1988-200310 XP002181114 -& JP 63 136054 A (FUJI PHOTO FILM CO LTD) , 8. Juni 1988 (1988-06-08)

## Beschreibung

Die Erfindung betrifft sogenannte Laser-kompatible NIR-Marker-Farbstoffe auf der Basis von Polymethinen zur Verwendung in optischen, insbesondere fluoreszenzoptischen, Bestimmungs- und Nachweisverfahren. Typische Verfahrensanwendungen beruhen auf der Reaktion von farbstoffmarkierten Antigenen, Antikörpern, Liganden oder DNA-Segmenten mit der jeweils komplementären Spezies.

Einsatzmöglichkeiten ergeben sich beispielsweise in der Medizin und der Pharmazie, in der Bio- und Materialwissenschaft, bei der Umweltkontrolle und dem Nachweis von in Natur oder Technik vorkommenden organischen und anorganischen Mikroproben, sind jedoch nicht auf die genannten Bereiche beschränkt.

Polymethine sind als NIR-Marker seit langem bekannt und zeichnen sich durch intensive, leicht in den NIR-Bereich verschiebbare Absorptionsmaxima aus (Fabian, J.; Nakazumi, H.; Matsuoka, M.: Chem. Rev. 1992, 92, 1197). Bei geeignetem Substituentenmuster und π-Elektronensystem fluoreszieren sie mit ausreichender Quantenausbeute auch im roten und nahem infraroten (NIR)-Bereich. Entsprechend finden diese Verbindungen breite Anwendung in verschiedenen Bereichen der Technik, als Sensibilisatoren in AgX-Materialien, als Laserfarbstoffe, als Quantenzähler, als Indikator-Farbstoffe in der Sensorik, als Licht-Absorber in beschreibbaren CD's und nicht zuletzt als Biomarker ("Near-Infrared Dyes for High Technology Applications", herausgegeben von Daehne, S.; Resch-Genger, U.; Wolfbeis, O.-S., Kluwer, Academic Publishers - Dordrecht/Boston/ London - 1998).

Die Anzahl der als Biomarker verwendeten Polymethine ist begrenzt. Breite kommerzielle Anwendung haben in diesem Sinne bisher nur das sich vom Astraphloxin (DE 410 487) abgeleitete Trimethin Cy3, bzw. das vinyloge Pentamethin Cy5 und das doppelt vinyloge Heptamethin Cy7 mit Absorptionsmaxima bei ca. 550 nm, ca. 650 nm und ca. 750 nm gefunden (US-PS 5 627 027). Darüber hinaus werden das polysulfonierte, vom kommerziellen Heptamethin "Indocyaningreen" bzw. "Cardio Green" abgeleitete Trimethin Cy3.5 und Pentamethin Cy5.5 angeboten (US-PS 5 569 766). In der Polymethinkette aliphatisch verbrückte Heptamethine wurden von Patonay entwickelt (US-PS 5 800 995). Charakteristisch für alle kommerziellen Biomarker sind die sich vom Inden bzw. Heteroinden (Fischer-Base) ableitenden terminalen Heteroaromaten. Werden methylsubstituierte Cycloimonium-Salze dieses Typs als terminale Polymethin-Bausteine verwendet, so ist es notwendig, mindestens fünf aufeinander folgende sp²-hybridisierte Kohlenstoffatome (Pentamethine) zwischen den Heterocyclen anzuordnen, um Absorptionsmaxima an der Grenze zum NIR-Bereich zu erzeugen.

Ein wesentlicher Nachteil der als Biomarker technisch genutzten NIR-Polymethine besteht darin, dass mit Verlängerung der Polymethinkette im steigenden Maße nucleophile bzw. elektrophile Angriffsmöglichkeiten auf die Kette gegeben sind, in deren Folge es zur Zerstörung des π-systems kommt. Weitere Nachteile dieser Marker-Farbstoffe bestehen in einer ungenügenden Photo- oder Lagerstabilität, in aufwendigen Synthese- und Reinigungsschritten, in geringen Absorptionskoeffizienten bzw. einer unbefriedigenden Fluoreszenzquantenausbeute sowie in unerwünschten Änderungen der optischen Eigenschaften in Gegenwart von Proteinen oder Nucleinsäureoligomeren bzw. nach Bindung an diese. Beispielsweise wurde eine Verminderung der Fluoreszenzquantenausbeute von Cy5 beim kovalenten Binden an verschiedene Albumine beschrieben (Oswald, B.; Patsenker, L.; Duschl, J.; Szmacinski, H.; Wolfbeis, O.S.; Terpetschnig, E.; Bioconjugate Chem. 1999, 10, 925-931).

Die Verwendung von Pyrylium- und Benzopyrylium-Heterocyclen bzw. den entsprechenden mesomeren Chromenen als terminale Endgruppen in Markerfarbstoffen in biologisch relevanten Systemen ist bisher nicht bekannt. Dies ist auf die extreme Hydrolyseempfindlichkeit dieser π-Mangelaromaten, vor allem in wässrig-basischem Milieu, zurückzuführen (H. Lietz, G. Haucke, P. Czerney, B. John, J. Prakt. Chem., 1996, 338, 725-730).

Telfer et al. (US-Patent 5 262 549) beschreiben symmetrische Trimethine auf der Basis von 2-alkyl-substituierten Benzopyryliumsalzen für die Verwendung als NIR-Absorber in polymeren Medien, wobei die Aggregationsneigung in diesen Medien reduziert ist.

Die Druckschrift DE,A,4,341,618 offenbart Indikatorfarbstoffe für den NIR-spektralbereich, die eine Benzpyran-2-yliden-Einheit aufweisen und zur pH-Wertmeßung sowie zur Schadstoffkonzentrationsbestimmung eingesetzt werden.

Der Erfindung liegt die Aufgabe zugrunde, NIR-Marker-Farbstoffe auf Polymethin-Basis mit hoher Photo- und Lagerstabilität sowie hoher Fluoreszenzausbeute zu schaffen, die auf möglichst einfache Weise durch Laserstrahlung im langwelligen sichtbaren oder im nahen IR-Spektralbereich, insbesondere mit Licht des Helium/Neon- oder Diodenlasers, zur Fluoreszenz angeregt werden können.

Die vorliegende Erfindung beschreibt Marker-Farbstoffe auf der Basis von unsymmetrischen Polymethinen, die eine substituierte ω-(Benz[b]pyran-4-yliden)alk-1-enyl)-Einheit der allgemeinen Struktur I 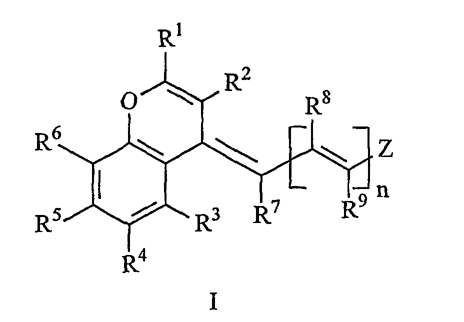 enthalten mit Z als substituierten Benzooxazol-, Benzothiazol-, 2,3,3-Trimethylindolenin-, 2,3,3-Trimethyl-4,5-benzo-3H-indolenin-, 2- und 4-Picolin-, Lepidin-, Chinaldin- sowie 9-Methylacridinderivaten der allgemeinen Formeln IIa oder IIb oder IIc 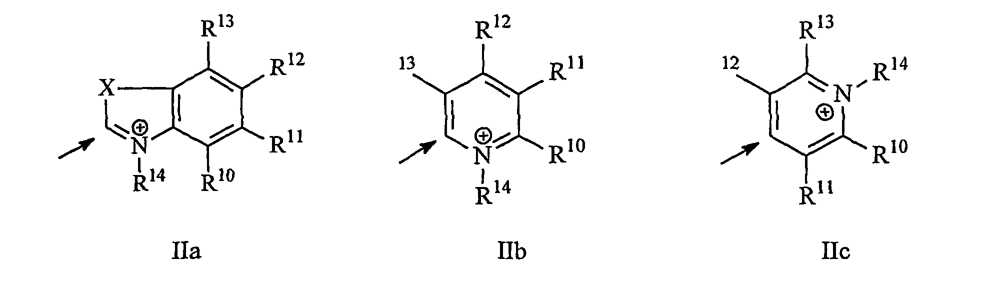 wobei
- X für ein Element aus der Gruppe O, S, Se oder das Strukturelement N-alkyl oder C(alkyl)₂ steht,
- n für die Zahlenwerte 1, 2 oder 3 steht,
- R¹ - R¹⁴ gleich oder unterschiedlich sind und Wasserstoff, ein oder mehrere Alkyl-, oder Aryl-, Heteroaryl- oder heterocycloaliphatische Reste, eine Hydroxy- oder Alkoxygruppe, eine alkylsubsitiuierte oder cyclische Aminfunktion sein können und/oder zwei ortho-ständige Reste, z. B. R¹⁰ und R¹¹, zusammen einen weiteren aromatischen Ring bilden können,
- mindestens einer der Substituenten R¹ - R¹⁴ einen solubilisierenden bzw. ionisierbaren bzw. ionisierten Substituenten, wie Cyclodextrin, Zucker, SO₃⁻, PO₃²⁻, COO⁻, oder NR₃⁺ darstellen kann, der die hydrophilen Eigenschaften dieser Farbstoffe bestimmt, wobei dieser Substituent auch über eine Spacergruppe am Markerfarbstoff angebunden sein kann,
- mindestens einer der Substituenten R¹ - R¹⁴ für eine reaktive Gruppe stehen kann, welche eine kovalente Verknüpfung des Farbstoffs mit den oben genannten Trägermolekülen ermöglicht, wobei dieser Substituent auch über eine Spacergruppe am Markerfarbstoff angebunden sein kann, und
- R¹ einen Substituenten darstellt, der in α-Position zum Pyran-Ring ein quartäres C-Atom aufweist. Beispiele für einen solchen Substituenten sind t-Butyl (-C(CH₃)₃) und Adamantyl (-C₁₀H₁₅ / Tricyclo[3.3.1.1^{3,7}]decyl).

In den Unteransprüchen 2-20 sind spezielle Ausführungsformen und Anwendungen zu den Marker-Farbstoffen aufgeführt.

Diese substituierten Indol-, Heteroindol-, Pyridin-, Chinolin- oder Acridinderivate der allgemeinen Formel I können als Farbstoffe zur optischen Markierung von organischen oder anorganischen Mikropartikeln, z. B. von Proteinen, Nucleinsäuren, DNA, Zuckern, biologischen Zellen, Lipiden, Pharmaka oder organischen bzw. anorganischen polymeren Trägermaterialien verwendet werden.

Die Markierung der Partikel kann dabei durch die Ausbildung von ionischen Wechselwirkungen zwischen den Markern der allgemeinen Formel I und den zu markierenden Materialien erfolgen.

Die gegenüber Nucleophilen aktivierten funktionellen Gruppen dieser Marker vermögen kovalent an eine OH-, NH₂- oder SH-Funktion zu koppeln. Somit entsteht ein System zur qualitativen oder quantitativen Bestimmung von organischen und anorganischen Materialien, wie den besagten Proteinen, Nukleinsäuren, DNA, Zuckern, biologische Zellen, Lipiden, Pharmaka oder organischen bzw. anorganischen Polymeren.

Diese Kopplungsreaktion kann in wässriger oder überwiegend wässriger Lösung und vorzugsweise bei Raumtemperatur durchgeführt werden. Dabei entsteht ein Konjugat mit fluoreszenten Eigenschaften.

Durch die Darstellung von nichtsymmetrischen Polymethinen, die einerseits als terminale Funktion einen leicht derivatisierbaren Heterocyclus vom Typ der Pyridin-, Chinolin-, Indol-, Heteroindol- bzw. Acridinderivate sowie andererseits einen neuartigen 6-Ringheterocyclus aufweisen, werden insbesondere nachfolgende Vorteile erreicht:

Bereits Trimethine absorbieren im Spektralbereich > 650 nm und zeigen eine gegenüber den bisher bekannten Polymethinen mit Absorptionsmaxima > 650 nm (Penta- und Hepta-methine) eine wesentlich verbesserte photochemische und thermische Stabilität.

Durch "molecular engineering" ist es möglich, Lage und Intensität der Absorptions- und Emissionsmaxima beliebig zu steuern und den Emissionswellenlängen unterschiedlicher Anregungslaser, vor allem NIR-Laserdioden, anzupassen.

Die Marker-Farbstoffe sind durch relativ einfache und in zwei Stufen durchzuführende Synthese herstellbar, mit welcher eine Vielzahl unterschiedlich funktionalisierter Farbstoffe, beispielsweise hinsichtlich der Gesamtladung des Farbstoffes und der Anzahl, Spezifität und Reaktivität der zur Immobilisierung genutzten aktivierten Gruppen, anwendungsspezifisch zur Verfügung gestellt werden kann.

Sowohl die Verbindungen der allgemeinen Formel I als auch davon abgeleitete Systeme (Konjugate) können in optischen, insbesondere fluoreszenzoptischen, qualitativen und quantitativen Bestimmungsverfahren zur Diagnostik von Zelleigenschaften, in Biosensoren (point of care-Messungen), Erforschung des Genoms und in Miniaturisierungstechnologien eingesetzt werden. Typische Anwendungen erfolgen in der Zytometrie und Zellsortierung, der Fluoreszenz-Korrelations-Spektroskopie (FCS), im Ultra-High-Troughput-Screening (UHTS), bei der multicolor Fluoreszenz-in-situ-Hybridisierung (FISH) und in Mikroarrays (Gen- und Proteinchips).

Dabei ist ein Mikroarray eine rasterartige Anordnung von auf mindestens einer Oberfläche immobilisierten Molekülen, die zum Studium von Rezeptor-Liganden-Wechselwirkungen verwendet werden können. Eine rasterartige Anordnung bedeutet mehr als zwei voneinander verschiedene Moleküle, welche sich innerhalb einer Fläche befinden und dort in unterschiedlichen, vorher definierten Regionen mit bekannter Position immobilisiert sind.

Ein Rezeptor ist ein Molekül, das eine Affinität zu einem gegebenen Liganden besitzt. Rezeptoren können natürlich vorkommende oder künstlich hergestellte Moleküle sein.

Rezeptoren können in reiner Form oder gebunden an andere Spezies eingesetzt werden. Rezeptoren können kovalent oder nichtkovalent entweder direkt oder durch bestimmte Kopplungsvermittler an einen Bindungspartner angeknüpft werden.

Beispiele für Rezeptoren, die durch diese Erfindung detektiert werden können, schließen Agonisten und Antagonisten für Zell-Membran-Rezeptoren, Toxine und andere Giftstoffe, virale Epitope, Hormone wie Opiate und Steroide, Hormonrezeptoren, Peptide, Enzyme, Enzymsubstrate, als Kofaktoren agierende Wirkstoffe, Lektine, Zucker, Oligonukleotide, Nukleinsäuren, Oligosaccharide, Zellen, Zellfragmente, Gewebefragmente, Proteine und Antikörper ein, sind aber nicht auf die angeführten Stoffe beschränkt.

Ein Ligand ist ein Molekül, das von einem bestimmten Rezeptor erkannt wird. Beispiele für Liganden, die durch diese Erfindung detektiert werden können, schließen Agonisten und Antagonisten für Zell-Membran-Rezeptoren, Toxine und andere Giftstoffe, virale Epitope, Hormone wie Opiate und Steroide, Hormonrezeptoren, Peptide, Enzyme, Enzymsubstrate, als Kofaktoren agierende Wirkstoffe, Lektine, Zucker, Oligonukleotide, Nukleinsäuren, Oligosaccharide, Proteine und Antikörper ein, sind aber nicht auf die angeführten Stoffe beschränkt.

Verwendung finden die Verbindungen der Formel I als auch davon abgeleitete Systeme in optischen, insb. fluoreszenzoptischen qualitativen und quantitativen Bestimmungsverfahren einschließlich Immuntests, Hybridisierungsverfahren, chromatographischen oder elektrophoretischen Verfahren und in Hoch-Durchsatz-Screening.

Die Erfindung soll nachstehend anhand von Ausführungsbeispielen und Zeichnungen näher erläutert werden.

In den dazugehörigen Zeichnungen zeigt
- Fig. 1: Strukturformel von Benzopyrylium-Salz 2a
- Fig. 2: Synthese und Strukturformel von Benzopyrylium-Salz 2b
- Fig. 3: Synthese und Strukturformel von Trimethin OB11 (DY-630)
- Fig. 4: Fluoreszenzspektren von OB15 (DY-635) in wäßriger Lösung und gebunden an Rinderserum-Albumin (BSA)
- Fig. 5: Fluoreszenzanregungsspektren von OB15 (DY-635) in wäßriger Lösung und gebunden an Rinderserum-Albumin (BSA)

### Ausführungsbeispiele:

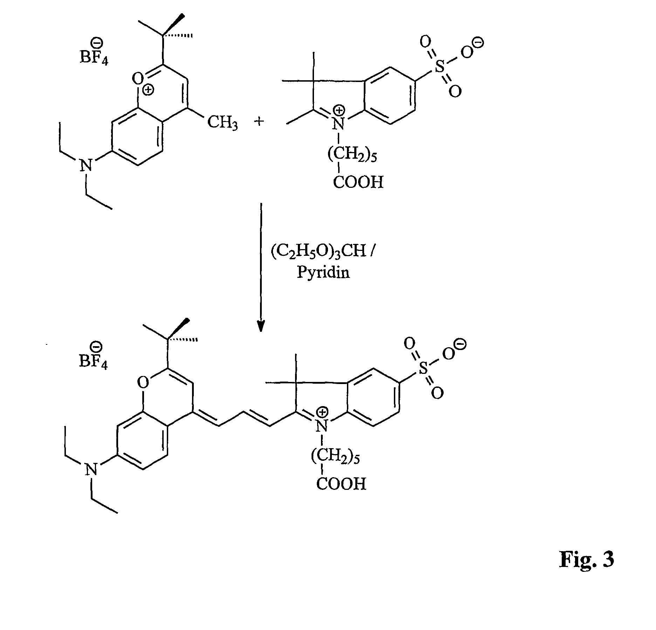

### 1. Vorschrift zur Darstellung von 11-(2,2-Dimethylethyl)-9-methyl-1 H,2H,3H,5H,6H,7H-pyrano[2,3-f]pyrido[3,2,1-ij]chinolin-12-ium tetrafluoroborat 2b (BS 28), vgl. Fig. 2:

Zu einer gekühlten Lösung von 7.3 g (0.0245 mol) 11-(2,2-Dimethylethyl)-1H,2H,3H,5H,6H,7H-pyrano[2,3-f] pyrido[3,2,1-ij]chinolin-9-on in 50 ml Ethylenglycoldimethylether werden tropfenweise 50 ml einer 1.0 molaren Lösung von Methylmagnesiumbromid in Dibutylether gegeben. Die Mischung wurde für 30 Minuten auf 40°C erwärmt. Nach Kühlung auf 0°C wurde 70 ml gesättigte NH₄Cl-Lösung und verdünnte Salzsäure zur Hydrolyse zugegeben. Die organische Phase wurde abgetrennt und mit 4 x 10 ml Diethylether extrahiert. Das Lösungsmittel wurde am Rotationsverdampfer entfernt und der ölige Rückstand in 20 ml Eisessig gelöst. Zugabe von 3 ml HBF₄ (48 - 50 %) und Verdünnen mit Diethylether führte zu einem Niederschlag, der abfiltriert und aus Eisessig umkristallisiert wird.
3.35 g (35 %) Ausbeute, 175-80°C Schmelzpunkt. - ¹H NMR (400 MHz, CDCl₃ + CF₃CO₂D): 1.43 (s, 9H), 1.90 (m, 2H), 2.06 (m, 2H), 2.67 (m, 2H), 2.92 (m, 2H), 3.35 (m, 2H), 3.57 (m, 2H), 3.95 (s, 3H), 6.90 (s, 1 H), 7.58 (s, 1H): - C₂₀H₂₆BF₄NO (383.24): ber. C 62.68, H 6.84, N 3.65, gef. C 63.06, H 6.72, N 3.48.

### 2. Allgemeine Vorschrift zur Darstellung der unsymmetrischen Trimethine OB 11, OB 14, OB 15 und OB 20:

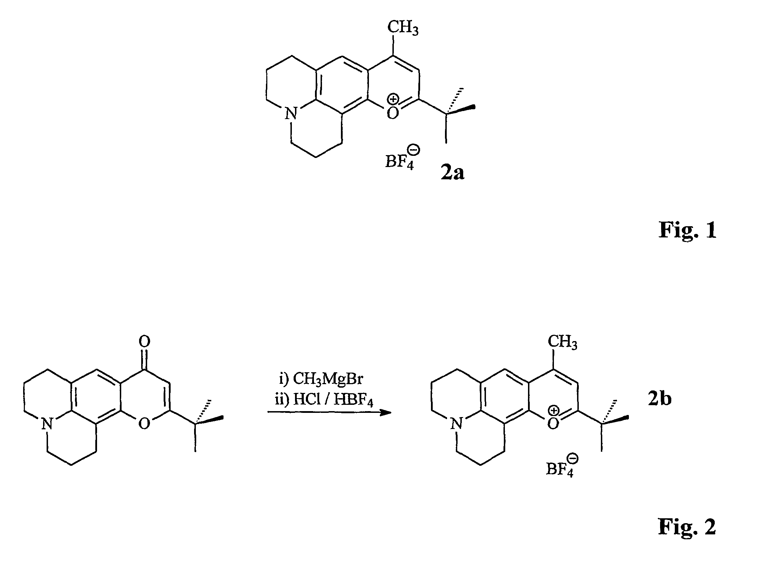

0.01 mol vom entsprechenden 4-Methyl-benzopyrylium-tetrafluoroborat der Formel 2a (BS4) oder 2b (BS28) (vgl. Fig. 1 und 2) und 0.01 mol methylenaktiver N-Heterocyclus wurden in 20 ml Acetanhydrid gelöst, mit 2,0 g Triethoxymethan und 5 ml Pyridin versetzt und ca. 10 min erhitzt. Nach dem Abkühlen der Lösung auf RT wurde das Farbstoffrohprodukt mit ca. 30 ml Diethylether ausgefällt. Der Niederschlag wurde abfiltriert und säulenchromatographisch gereinigt.

### 3. 1-(5-Carboxypentyl)-3,3-dimethyl-2-[3-(7-N,N-diethylamino-2-(1,1-dimethylethyl)-4H-benzopyran-4-yliden)-1-propenyl]-3H-indolium-5-sulfonat OB 11 (DY-630):

0.01 mol 2a und 0.01 mol 1-(5-Carboxypentyl)-2,3,3-trimethyl-3H-indolium-5-sulfonat wurden entsprechend der allgemeinen Vorschrift 1 umgesetzt, vgl. Fig.3. Säulenchromatographie: SiO₂, Eluent Ethanol. 3.2 g (50 %) Ausbeute, 280-82°C Schmelzpunkt. - ¹H NMR (400 MHz, DMSO-d6): 1.10 - 1.86 (m, 27H), 2.16 (m, 2H), 3.54 (m, 4H), 4.13 (m, 2H), 6.58 (d, 1 H), 6.74 (s, 1 H), 6.97 (s, 1 H), 7.06 (d, 1 H), 7.14 (d, 1 H), 7.36 (d, 1 H), 7.68 (d, 1 H), 7.78 (s, 1 H), 8.08 (d, 1 H), 8.32 (t, 1H) - ¹³C NMR (100 MHz, DMSO-d6): 12.30, 21.51, 24,32, 25.66, 26.56, 27.55, 34.07, 36.37, 43.72, 44.22, 48.87, 96.36, 99.40, 104.11, 109.85, 110.28, 112.48, 113.27, 119.66, 126.09, 140.23, 141.81, 145.59, 147.09, 162.14, 172.33, 174.64 - MS (FAB in dmba): 657 (M+Na+), 635 (M+H⁺), 391, 359, 258, 257 - C₃₆H₄₆N₂O₆S (634.83): ber. C 68.11, H 7.30, N 4.41, gef. C 68.25, H 7.33, N 4.39.

### 4. 1-(3-Hydroxypropyl)-4-[3-(7-N,N-diethylamino-2-(1,1-dimethylethyl)-4H-benzopyran-4-yliden)-1-propenyl]-chinolinium-tetrafluoroborat OB 14:

0.01 mol 2a und 0.01 mol 1-(3-Hydroxypropyl)-4-methylchinolinium-iodid wurden entsprechend der allgemeinen Vorschrift 1 umgesetzt.
Säulenchromatographie: SiO₂, Eluent Toluol/Ethanol 1/1. 2.4 g (42 %) Ausbeute, 162-64°C Schmelzpunkt. - ¹H NMR (400 MHz, CDCl₃): 1.17 (t, 6H), 1.32 (s, 9H), 2.14 (m, 2H), 2.25 (s, 1 H), 3.39 (q, 4H), 3.71 (m, 2H), 4.89 (m, 2H), 6.31 (d, 1 H), 6.56 (s, 1 H), 6.62 (m, 2H), 7.01 (d, 1 H), 7.60 (t, 1 H), 7.67 (d, 1 H), 7.77 (d, 1 H), 7.84 (t, 1 H), 7.93 (d, 1 H), 8.12 (t, 1 H), 8.31 (d, 1 H), 9.27 (d, 1H). - ¹³C NMR (100 MHz, CDCl₃) : 12.52, 28.08, 32.01, 36.20, 44.61, 52.53, 57.52, 97.05, 97.94, 109.58, 109.94, 110.91, 111.77, 113.61, 117.72, 124.79, 125.38, 125.50, 127.13, 133.64, 137.96, 140.92, 142.20, 144.96, 150.87, 151.74, 155.40, 167.12 - MS (FAB in dmba): 483 (M⁺) - C₃₂H₃₉BF₄N₂O₂ (570.48): ber. C 67.37, H 6.89, N 4.91, gef. C 67.30, H 6.92, N 4.89.

### 5. 1-(5-Carboxypentyl)-3,3-dimethyl-2-[3-(11-(2,2-dimethylethyl)-1 H,2H,3H,5H,6H,7H-pyrano[2,3-f]pyrido[3,2,1-ij]chinolin-9-yliden)-1-propenyl]-3H-indolium-5-sulfonat OB 15 (DY-635):

0.01 mol 2b und 0.01 mol 1-(5-Carboxypentyl)-2,3,3-trimethyl-3H-indolium-5-sulfonat wurden entsprechend der allgemeinen Vorschrift 1 umgesetzt.

Säulenchromatographie: SiO₂, Eluent Ethanol. 2.9 g (44 %) Ausbeute, >300°C Schmelzpunkt. - ¹H NMR (250 MHz, DMSO-d6): 1.10 - 1.56 (m, 19H), 1.91 (m, 4H), 2.08 (m, 4H), 2.83 (m, 4H), 3.38 (m, 4H), 4.03 (m, 2H), 6.45 (d, 1 H), 6.97 (s, 1 H), 7.13 (d, 1 H), 7.26 (d, 1 H), 7.62 (d, 1 H), 7.73 (s, 1 H), 7.78 (s, 1 H), 8.23 (t, 1H) - ¹³C NMR (62 MHz, DMSO-d6): 19.40, 20.43, 24.86, 25.98, 26.61, 27.16, 27.76, 27.85, 28.94, 35.17, 36.71, 43.40, 48.45, 49.04, 49.63, 99.24, 102.90, 105.09, 109.69, 110.03, 112.96, 119.71, 121.85, 123.50, 139.89, 142.18, 144.84, 145.76, 148.56, 148.86, 151.59, 170.08, 171.37 - MS (ESI): 681 (M+Na⁺), 659 (M+H⁺), 352 - C₃₈H₄₆N₂O₆S (658.12): ber. C 69.27, H 7.34, N 4.25, gef. C 69.20, H 7.37, N 4.29.

### 6. 1-(5-Carboxypentyl)-4-[3-(7-N,N-diethylamino-2-(1,1-dimethylethyl)-4H-benzo-pyran-4-yliden)-1-propenyl]-chinolinium-6-sulfonat OB 20:

0.01 mol 2a und 0.01 mol 1-(5-Carboxypentyl)-4-methyl-chinolinium-6-sulfonat wurden entsprechend der allgemeinen Vorschrift 1 umgesetzt.
Säulenchromatographie: SiO₂, Eluent Ethanol. 2.1 g (35 %) Ausbeute, >300°C Schmelzpunkt. - C₃₅H₄₂N₂O₆S (618.76): ber. C 67.93, H 6.84, N 4.53, gef. C 67.73, H 6.93, N 4.29.

### 7. Darstellung des NHS-Esters von OB 11 (DY-630) mit N-Hydroxysuccinimid (NHS)/N,N'-Dicyclo-hexylcarbodiimid (DCC)

15 mg OB 11 (DY-630), 14 mg DCC und 4 mg NHS wurden in 1 ml trockenem DMF gelöst. Dann gab man 10 µl Triethylamin zu. Das Reaktionsgemisch wurde 24 Stunden bei Raumtemperatur gerührt und anschließend filtriert. Dann wurde das Lösungsmittel abgezogen, der Rückstand mit Ether gewaschen. Diese Reaktion verlief quantitativ.

### 8. Darstellung des NHS-Esters von OB 15 (DY-635) mit N-Hydroxysuccinimid (NHS)/N,N'-Dicyclo-hexylcarbodiimid (DCC)

Es wurde analog zum Beispiel Nr. 7 verfahren. Auch diese Reaktion verlief quantitativ.

### 9. Anregungs- und Emissionsspektren von 1-(5-Carboxypentyl)-3,3-dimethyl-2-[3-(11-(2,2-dimethylethyl)-1H,2H,3H,5H,6H,7H-pyrano[2,3-f]pyrido[3,2,1-ij]chinolin-9-yliden)-1-propenyl]-3H-indolium-5-sulfonat OB15 (DY-635)

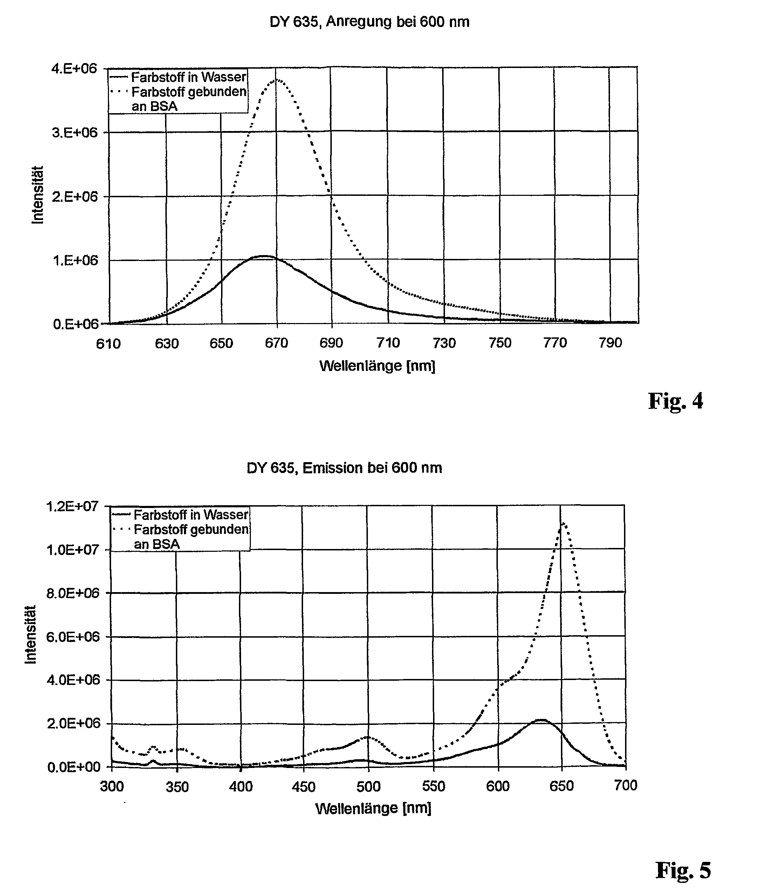

Die Abbildungen in Fig. 4 zeigen die Emissions- und in Fig.5 die Anregungsspektren von 1-(5-Carboxypentyl)-3,3-dimethyl-2-[3-(11-(2,2-dimethylethyl)-1 H,2H,3H,5H,6H,7H-pyrano [2,3-f]pyrido[3,2,1-ij]chinolin-9-yliden)-1-propenyl]-3H-indolium-5-sulfonat in Wasser und nichtkovalent gebunden an Rinder-Serum-Albumin (BSA), wobei jeweils das intensivere Spektrum das BSA-Konjugat kennzeichnet. Beide Farbstofflösungen waren bei diesen Messungen gleich konzentriert.

### 10. Allgemeine Vorschrift zur Markierung von Proteinen

Die Proteinmarkierungen wurden in einem 50 mM Bicarbonatpuffer (pH 9,0) ausgeführt. Es wurde eine Stammlösung mit 0.5 mg Reaktivfarbstoff (z. B. OB 11-NHS-ester, M=732 g- mol⁻¹) in 100 µl DMF hergestellt. Das Protein, z.B. Avidin (M=66000 g- mol⁻¹) wurde portionsweise zu je 1 mg in 200 ml Bicarbonatpuffer gelöst, worauf verschiedene Volumina der ggf. verdünnten Farbstoff-Stammlösung zu den einzelnen Protein-Aliquots hinzugegeben wurden. Dann wurden die Reaktionsgemische 1 - 2 Stunden bei Raumtemperatur gerührt. Der freie Farbstoff wurde von den markierten Proteinen durch Gelchromatographie (Sephadex G25 medium, Eluent PBS pH 7.2 22mM) abgetrennt.

## Patentansprüche

1. Marker-Farbstoffe auf der Basis von unsymmetrischen Polymethinen, die eine substituierte ω-(Benz[b]pyran-4-yliden)alk-1-enyl)-Einheit der allgemeinen Struktur I 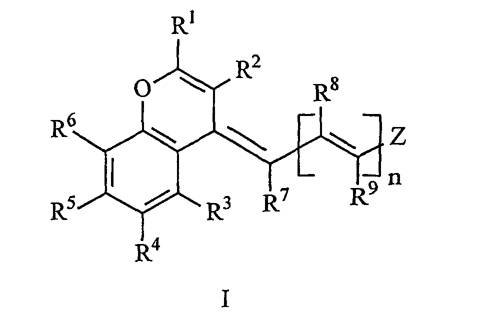 enthalten mit Z als substituierten Benzooxazolium-, Benzothiazolium-, 3,3-Dimethylindolium-, 3,3-Dimethyl-4,5-benzo-3*H*-indolium-, 2- und 4-Pyridinium-, 2- und 4-Chinolinium- sowie 9-Acridiniumderivaten der allgemeinen Formeln IIa oder IIb oder IIc 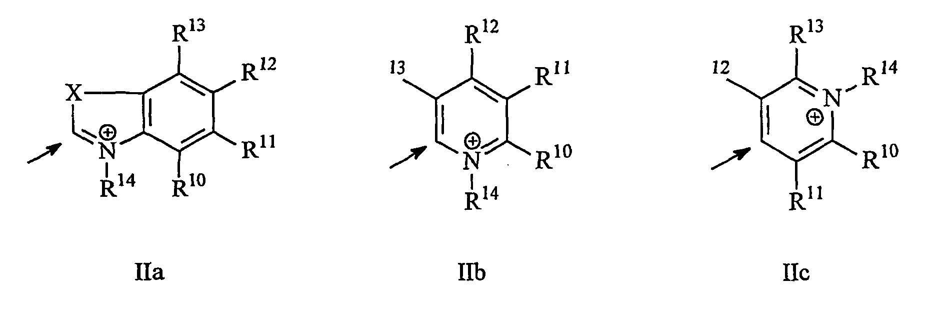 wobei
- X für ein Element aus der Gruppe O, S, Se oder das Strukturelement N-alkyl oder C(alkyl)₂ stehen,
- n für die Zahlenwerte 0, 1, 2 oder 3 steht,
- R¹ - R¹⁴ gleich oder unterschiedlich sind und Wasserstoff, ein oder mehrere Alkyl-, oder Aryl-, Heteroaryl- oder heterocycloaliphatische Reste, eine Hydroxy- oder Alkoxygruppe, eine alkylsubsituierte oder cyclische Aminfunktion sein können und/oder zwei ortho-ständige Reste, z. B. R¹⁰ und R¹¹, zusammen einen weiteren aromatischen Ring bilden können,
- mindestens einer der Substituenten R¹ - R¹⁴ einen solubilisierenden bzw. ionisierbaren bzw. ionisierten Substituenten, wie Cyclodextrin, Zucker, SO₃⁻, PO₃²⁻, COO⁻, oder NR₃⁺ darstellen kann, der die hydrophilen Eigenschaften dieser Farbstoffe bestimmt, wobei dieser Substituent auch über eine Spacergruppe am Markerfarbstoff angebunden sein kann,
- mindestens einer der Substituenten R¹ - R¹⁴ für eine reaktive Gruppe stehen kann, welche eine kovalente Verknüpfung des Farbstoffs mit einem anderen Molekül ermöglicht, wobei dieser Substituent auch über eine Spacergruppe am Markerfarbstoff angebunden sein kann, und
- R¹ einen Substituenten darstellt, der in α-Position zum Pyran-Ring ein quartäres C-Atom aufweist, wobei die Substituenten R¹ und R² auch ein aliphatisches bzw. substituiertes aliphatisches Ringsystem bilden können.

2. Laser-kompatible NIR-Marker-Farbstoffe gemäß Anspruch 1, **dadurch gekennzeichnet, dass** die reaktive Gruppe aus folgenden Funktionalitäten ausgewählt ist: Isothiocyanate, Isocyanate, Monochlortriazine, Dichlortriazine, Aziridine, Sulfonylhalogenide, Carbonsäurechloride, *N*-Hydroxysuccinimidester, Imido-Ester, Glyoxal oder Aldehyd für Amin- und Hydroxy-Funktionen bzw. Maleimide oder lodacetamide für Thiol-Funktionen sowie Phosphoramidite für die Markierung der DNA oder RNA oder deren Bruchstücke.

3. Laser-kompatible NIR-Marker-Farbstoffe gemäß Anspruch 1-2, **dadurch gekennzeichnet, dass** die reaktive Gruppe über Spacer-Gruppen der allgemeinen Struktur -(CH₂)ₘ- am eigentlichen Chromophor gebunden ist, wobei m Werte von 1 bis 18 annehmen kann.

4. Laser-kompatible NIR-Marker-Farbstoffe gemäß Anspruch 1-3, **dadurch gekennzeichnet, dass** die Struktureinheit =CR⁸- auch eine Verbrückung über vier-, fünf- und sechsgliedrige Ringsysteme beinhaltet, wobei sich an dieser auch reaktive Gruppen befinden und die Substituenten A - G die gleiche Funktionalität wie die Substituenten R¹ - R¹⁴ besitzen können.

5. Laser-kompatible NIR-Marker-Farbstoffe gemäß Anspruch 4, **dadurch gekennzeichnet, dass** die Struktureinheit =CR⁸- für 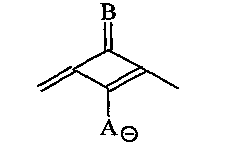 steht.

6. Laser-kompatible NIR-Marker-Farbstoffe gemäß Anspruch 4, **dadurch gekennzeichnet, dass** die Struktureinheit =CR⁸- für 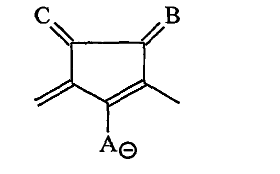 steht.

7. Laser-kompatible NIR-Marker-Farbstoffe gemäß Anspruch 4, **dadurch gekennzeichnet, dass** die Struktureinheit =CR⁸- für 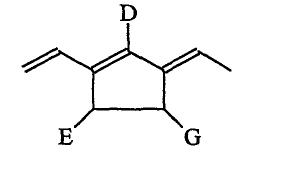 steht.

8. Laser-kompatible NIR-Marker-Farbstoffe gemäß Anspruch 4, **dadurch gekennzeichnet, dass** die Struktureinheit =CR⁸- für 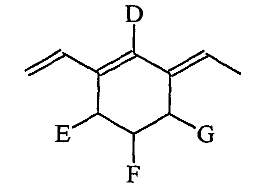 steht.

9. Laser-kompatible NIR-Marker-Farbstoffe gemäß den Ansprüchen 1-8, **dadurch gekennzeichnet, dass** die Substituenten A - G die gleiche Gestalt wie die Substituenten R¹ bis R¹⁴ annehmen können oder für O, S, C(CN)₂ bzw. N-R stehen, wobei R in N-R für einen aliphatischen oder aromatischen bzw. einem reaktiven aliphatischen oder aromatischen Rest, wie (CH₂)ₙCOOH oder (CH₂)ₙNH₂, stehen kann.

10. Laser-kompatible NIR-Marker-Farbstoffe gemäß den Ansprüchen 1-8, **dadurch gekennzeichnet, dass** der Substituent D für Cl, einen Alkyl-, Alkoxy-, Cycloalkoxy-, Phenolat, Alkylmercapto oder Phenylmercapto-Rest steht, an dem gegebenenfalls reaktive Substituenten entsprechend den Substituenten R¹ bis R¹⁴ angebracht sind.

11. Laser- kompatible NIR-Marker-Farbstoffe gemäß Anspruch 1, **dadurch gekennzeichnet, dass** der Substituent R¹ für 1,1-Dimethylethyl, 6,6-Dimethylbicyclo-[3,1,1]hept-2-en-2-yl, Bicyclo[2,2,1]-hept-2-en-5-yl, 3,3-Dimethylbut-1-en-1-yl oder Adamant-1-yl (-C₁₀H₁₅ / Tricyclo[3.3.1.1^{3,7}] decyl) steht.

12. Laser- kompatible NIR-Marker-Farbstoffe gemäß Anspruch 1, **dadurch gekennzeichnet, dass** der Substituent R⁵ für einfach oder doppelt alkyliertes Amin steht, wobei über den Aminostickstoff Alkyl - und Alkenylverbrückungen mit den Substituenten R⁴ und R⁶ möglich sind.

13. Laserkompatible NIR-Farbstoffe gemäß den Ansprüchen 1-12, **dadurch gekennzeichnet, dass** der ω-(Benz[b]pyran-4-yliden)alk-1-enyl)-Teil der Farbstoffe folgende Struktur Ia, Ib, Ic oder Id besitzt 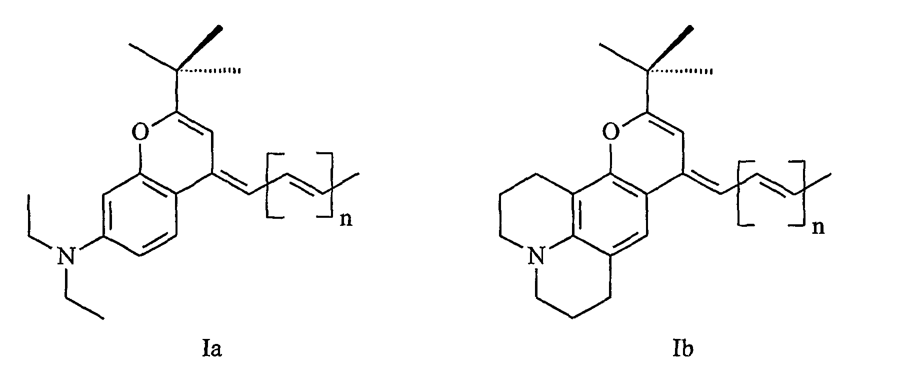 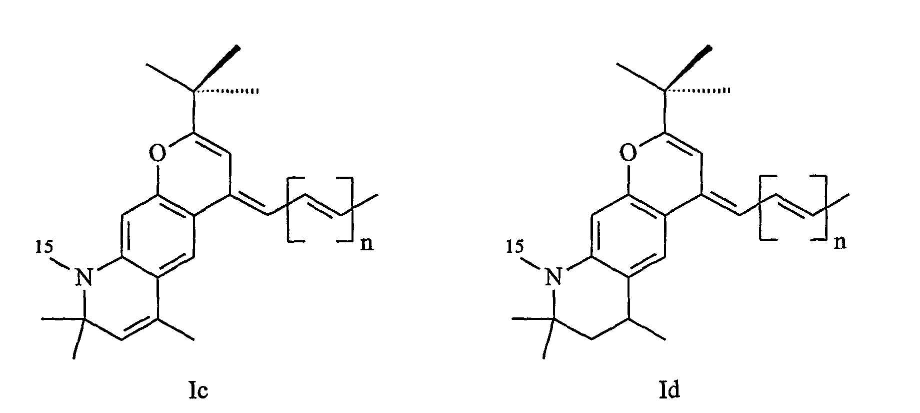 wobei der Substituent R¹⁵ die gleichen Funktionalitäten wie die Substituenten R¹ bis R¹⁴ besitzen kann.

14. Einen Laser-kompatiblen Markerfarbstoff gemäß den Ansprüchen 1-13, dergestalt dass
a. R², R³, R⁴, R⁶, R⁷, R⁸, R⁹ gleich H, R¹ gleich 1,1-Dimethylethyl, R⁵ gleich N,N-Diethylamino, n=1, Z gleich Ila mit R¹⁰, R¹¹, R¹³ gleich H, R¹² gleich SO₃⁻ und R¹⁴ gleich 5-Carboxypent-1-yl, namentlich 1-(5-Carboxypent-1-yl)-3,3-dimethyl-2-[3-(7-N,N-diethylamino-2-(1,1-dimethylethyl)-4H-benzopyran-4-yliden)-1-propen-1-yl]-3H-indolium-5-sulfonat (OB11)
b. R², R³, R⁷, R⁸, R⁹ gleich H, R⁴, R⁵, R⁶ zusammen einen Julolidin-Ring bilden, R¹ gleich 1,1-Dimethylethyl, n=1, Z gleich gleich Ila mit R¹⁰, R¹¹, R¹³ gleich H, R¹² gleich SO₃⁻ und R¹⁴ gleich 5-Carboxypent-1-yl, namentlich 1-(5-Carboxypentyl)-3,3-dimethyl-2-[3-(11-(2,2-dimethylethyl)-1 H,2H,3H,5H,6H,7H-pyrano[2,3-f]pyrido [3,2,1-ij]chinolin-9-yliden)-1-propenyl]-3H-indolium-5-sulfonat (OB15)
c. R², R³, R⁴, R⁶, R⁷, R⁸, R⁹ gleich H, R¹ gleich 1,1-Dimethylethyl, R⁵ gleich N,N-Diethylamino, n=1, Z gleich IIc mit R¹⁰, R¹¹ gleich H, R¹² und R¹³ zusammen einen ankondensierten Ring der Struktur CH=C(SO₃⁻)-CH=CH bilden und R¹⁴ gleich 5-Carboxypent-1-yl, namentlich 1-(5-Carboxypent-1-yl)-4-[3-(7-N,N-diethylamino-2-(1,1-dimethylethyl)-4H-benzopyran-4-yliden)-1-propen-1-yl]-chinolinium-6-sulfonat (OB20)
d. R², R³, R⁴, R⁶, R⁷, R⁸, R⁹ gleich H, R¹ gleich 1,1-Dimethylethyl, R⁵ gleich N,N-Diethylamino, n=1, Z gleich IIc mit R¹⁰, R¹¹ gleich H, R¹² und R¹³ zusammen einen ankondensierten Ring der Struktur CH=CH-CH=CH bilden und R¹⁴ gleich 3-Hydroxyprop-1-yl, namentlich 1-(3-Hydroxyprop-1-yl)-4-[3-(7-N,N-diethylamino-2-(1, 1-dimethylethyl)-4H-benzopyran-4-yliden)-1-propen-1-yl]-chinolinium-Perchlorat (OB 14).

15. Verwendung der substituierten Pyran-Derivate der allgemeinen Formel I gemäß Anspruch 1 als Farbstoffe zur optischen Markierung von Proteinen, Nukleinsäuren, Oligomeren, DNA, RNA, biologischen Zellen, Lipiden, Polymeren, Pharmaka oder Polymerpartikeln.

16. System zur qualitativen oder quantitativen Bestimmung von Proteinen, Nukleinsäuren, Oligomeren, DNA, RNA, biologischen Zellen, Lipiden, Polymeren, Pharmaka oder Polymerpartikeln, **dadurch gekennzeichnet, dass** die funktionellen Gruppen der Verbindungen nach Anspruch 1 bis 14 kovalent an eine OH-, NH₂- oder SH-Funktion der zu bestimmenden Substanzen gekoppelt werden.

17. System nach Anspruch 16, **dadurch gekennzeichnet, dass** die Kopplungsreaktion in wässriger Lösung durchgeführt wird.

18. System nach Anspruch 16 oder 17, **dadurch gekennzeichnet, dass** die kovalent gekoppelte Verbindung fluoreszierende Eigenschaften aufweist.

19. Verwendung der Verbindungen und Systeme nach Anspruch 1 bis 18 in optischen, insbesondere fluoreszenzoptischen qualitativen und quantitativen Bestimmungsverfahren einschließlich Immuntests, Hybridisierungsverfahren, chromatographischer oder elektrophoretischer Verfahren und des Hoch-Durchsatz-Screenings.

20. Verwendung der in den Ansprüchen 1 bis 18 aufgeführten Verbindungen und Systeme zur Analyse von Rezeptor-Liganden-Wechselwirkungen auf einem Mikroarray.

## Claims

1. Marker dyes based on unsymmetrical polymethines which contain a substituted ω-(benz[b]pyran-4-ylidene)alk-1-enyl) unit of the general structure (I) 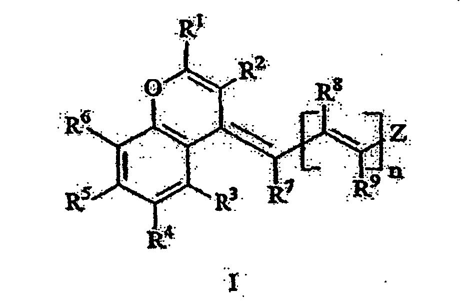 contain with Z as substituted benzooxazolium, benzothiazolium, 3,3-dimethylindolium, 3,3-dimethyl-4,5-benzo-3H-indolium, 2- and 4-pyridinium, 2- and 4-quinolinium and 9-acridinium derivatives of the general formulae IIa or IIb or IIc 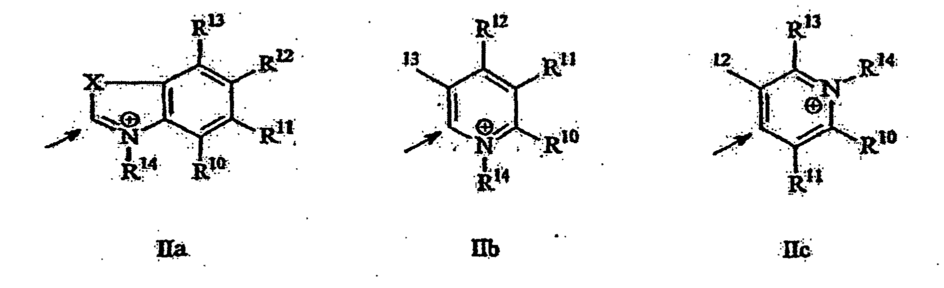 where
- X stands for an element from the group O, S, Se or the structural element N-alkyl or C(alkyl)₂,
- n stands for the numerical values 0, 1, 2 or 3,
- R¹ - R¹⁴ are equal or different and can be hydrogen, one or more alkyl, or aryl, heteroaryl or heterocycloaliphatic radicals, a hydroxy or alkoxy group, an alkyl-substituted or cyclic amine function and/or two radicals in the *ortho* position, for example R¹⁰ and R¹¹, can together form a further aromatic ring,
- at least one of the substituents R¹ - R¹⁴ can be a solubilising or ionisable or ionised substituent, such as cyclodextrin, sugar, SO₃⁻, PO₃²⁻, COO⁻ or NR₃⁺, which determines the hydrophilic properties of these dyes, wherein this substituent can also be bound to the marker dye by way of a spacer group,
- at least one of the substituents R¹ - R¹⁴ can stand for a reactive group which facilitates a covalent linking of the dye to another molecule, wherein this substituent can also be bound to the marker dye by way of a spacer group, and
- R¹ is a substituent which has a quaternary C atom in the a position relative to the pyran ring, wherein the substituents R¹ and R² can also form an aliphatic or a substituted aliphatic ring system.

2. Laser-compatible NIR marker dyes as claimed in Claim 1, **characterised in that** the reactive group is selected from among the following functionalities: isothiocyanates, isocyanates, monochlorotriazines, dichlorotriazines, aziridines, sulphonylhalogenides, carboxylic acid chlorides, N-hydroxysuccinimide esters, imido esters, glyoxal or aldehyde for amine and hydroxy functions or maleimides or iodoacetamides for thiol functions and phosporamidites for the marking of DNA or RNA or fragments thereof.

3. Laser-compatible NIR marker dyes as claimed in Claims 1 to 2, **characterised in that** the reactive group is bound to the actual chromophore by way of spacer groups of the general structure -(CH₂)ₘ, where m can have values from 1 to 18.

4. Laser-compatible NIR marker dyes as claimed in Claims 1 to 3, **characterised in that** the structural unit =CR⁸- also contains a bridge over four-, five- and six-membered ring systems, wherein reactive groups are also located thereon and the substituents A - G can have the same functionality as the substituents R¹ - R¹⁴.

5. Laser-compatible NIR marker dyes as claimed in Claim 4, **characterised in that** the structural unit =CR⁸- stands for

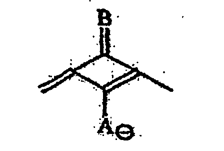

6. Laser-compatible NIR marker dyes as claimed in Claim 4, **characterised in that** the structural unit =CR⁸- stands for

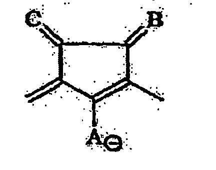

7. Laser-compatible NIR marker dyes as claimed in Claim 4, **characterised in that** the structural unit =CR⁸- stands for

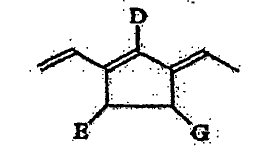

8. Laser-compatible NIR marker dyes as claimed in Claim 4, **characterised in that** the structural unit =CR⁸- stands for

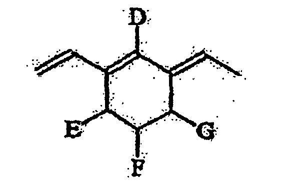

9. Laser-compatible NIR marker dyes as claimed in Claims 1 to 8, **characterised in that** the substituents A - G can assume the same configuration as the substituents R¹ to R¹⁴ or stand for O, S, C(CN)₂ or N-R, where R in N-R can stand for an aliphatic or aromatic or a reactive aliphatic or aromatic radical, such as (CH₂)ₙCOOH or (CH₂)ₙNH₂.

10. Laser-compatible NIR marker dyes as claimed in Claims 1 to 8, **characterised in that** the substituent D stands for Cl, an alkyl, alkoxy, cycloalkoxy, phenolate, alkylmercapto or phenylmercapto radical to which reactive substituents corresponding to the substituents R¹ to R¹⁴ are possisbly attached.

11. Laser-compatible NIR marker dyes as claimed in Claim 1, **characterised in that** the substituent R¹ stands for 1,1-dimethylethyl, 6,6-dimethylbicyclo-[3,1,1]-hept-2-en-2-yl, bicyclo[2,2,1]-hept-2-en-5-yl, 3,3-dimethylbut-1-en-1-yl or adamant-1-yl (-C₁₀H₁₅/tricyclo[3.3.1.1^{3,7}]decyl).

12. Laser-compatible NIR marker dyes as claimed in Claim 1, **characterised in that** the substituent R⁵ stands for a singly or doubly alkylated amine, wherein alkyl and alkenyl bridges with the substituents R⁴ and R⁶ are possible over the aminonitrogen.

13. Laser-compatible NIR marker dyes as claimed in Claims 1 to 12, **characterised in that** the ω-(benz[b]pyran-4-ylidene)alk-1-enyl) part of the dyes has the following structure Ia, Ib, Ic or Id 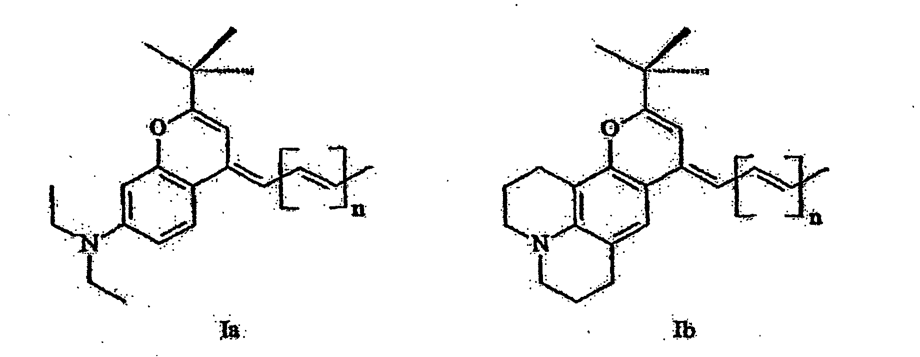 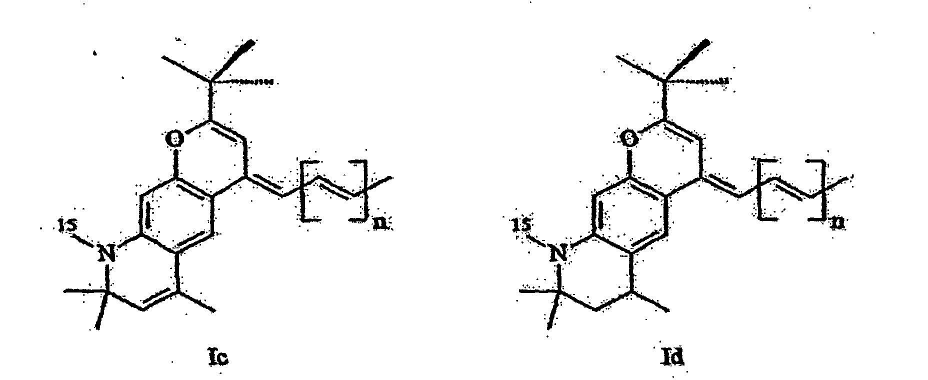 where the substituent R¹ can have the same functionalities as the substituents R¹ to R¹⁴.

14. Laser-compatible NIR marker dye as claimed in Claims 1 to 13, such that
a. R², R³, R⁴, R⁶, R⁷, R⁸, R⁹ are equal to H, R¹ is equal to 1,1-dimethylethyl, R⁵ is equal to N,N-diethylamino, n=1, Z is equal to IIa with R¹⁰, R¹¹, R¹³ equal to H, R¹² is equal to SO₃⁻ and R¹⁴ is equal to 5-carboxypent-1-yl, namely 1-(5-carboxypent-1-yl)-3,3-dimethyl-2-[3-(7-N,N-diethylamino-2-(1,1-dimethylethyl)-4H-benzopyran-4-ylidene)-1-propen-1-yl]-3H-indolium-5-sulphonate (OB 11),
b. R², R³, R⁷, R⁸, R⁹ are equal to H, R⁴, R⁵, R⁶ together form a julolidine ring, R¹ is equal to 1,1-dimethylethyl, n=1, Z is equal to with R¹⁰, R¹¹, R¹³ equal to H, R¹² is equal to SO₃⁻ and R¹⁴ is equal to 5-carboxypent-1-yl, namely 1-(5-carboxypentyl)-3,3-dimethyl-2-(3-(11-(2,2-dimethylethyl)-1H,2H,3H,5H,6H,7H-pyrano[2,3-f]pyrido[3,2,1-ij]quinolin-9-ylidene)-1-propenyl]-3H-indolium-5-sulphonate (OB 15),
c. R², R³, R⁶, R⁷, R⁸, R⁹ are equal to H, R¹ is equal to 1,1-dimethylethyl, R⁵ is equal to N,N-diethylamino, n=1, Z is equal to IIc with R¹⁰, R¹¹ equal to H, R¹² and R¹³ together form a ring which is condensed on and has the structure CH=C(SO₃⁻)-CH=CH, and R¹⁴ is equal to 5-carboxypent-1-yl, namely 1-(5-carboxypent-1-yl)-4-[3-(7-N,N-diethylamino-2-(1,1-dimethylethyl)-4H-benzopyran-4-ylidene)-1-propen-1-yl]-quinolinium-6-sulphonate (OB 20),
d. R², R³, R⁶, R⁷, R⁸, R⁹ are equal to H, R¹ is equal to 1,1-dimethylethyl, R⁵ is equal to N,N-diethylamino, n=1, Z is equal to IIc with R¹⁰, R¹¹ equal to H, R¹² and R¹³ together form a ring which is condensed on and has the structure CH=CH-CH=CH, and R¹⁴ is equal to 3-hydroxyprop-1-yl, namely (1-(3-hydroxyprop-1-yl)-4-[3-(7-N,N-diethylamino-2-(1,1-dimethylethyl)-4H-benzopyran-4-ylidene)-1-propen-1-yl]-quinolinium-perchlorate (OB 14).

15. Use of the substituted pyran derivatives of the general formula I as claimed in Claim 1 as dyes for the optical marking of proteins, nucleic acids, oligomers, DNA, RNA, biological cells, lipids, polymers, drugs or polymer particles.

16. System for the qualitative or quantitative determination of proteins, nucleic acids, oligomers, DNA, RNA, biological cells, lipids, polymers, drugs or polymer particles, **characterised in that** the functional groups of the compounds as claimed in Claims 1 to 14 are coupled covalently to an OH, NH₂ or SH function of the substances to be determined.

17. System as claimed in Claim 16, **characterised in that** the coupling reaction is carried out in an aqueous solution.

18. System as claimed in Claim 16 or 17, **characterised in that** the covalently coupled compound has fluorescent properties.

19. Use of the compounds and systems as claimed in Claims 1 to 18 in optical, particularly fluorescence optical qualitative and quantitative determination methods including immune tests, hybridisation methods, chromatographic or electrophoretic methods and high-throughput screenings.

20. Use of the compounds and systems set out in Claims 1 to 18 for the analysis of receptor/ ligands interactions on a microarray.

## Revendications

1. Colorants marqueurs à base de polyméthines asymétriques, qui contiennent une unité ω-(benzo[b]pyrann-4-ylidène)alc-1-ényle substituée, de la formule générale I: 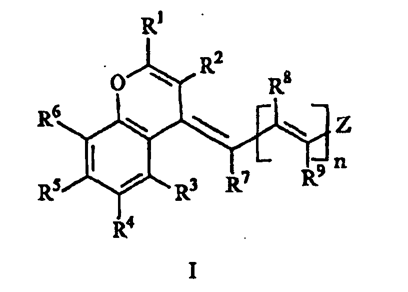 avec Z comme dérivés benzooxazolium, benzothiazolium, 3,3-diméthylindolium, 3,3-diméthyl-4,5-benzo-3H-indolium, 2- et 4-pyridinium, 2- et 4-quinoléinium ainsi que 9-acridinium substitués de formules générales IIa ou IIb ou IIc : 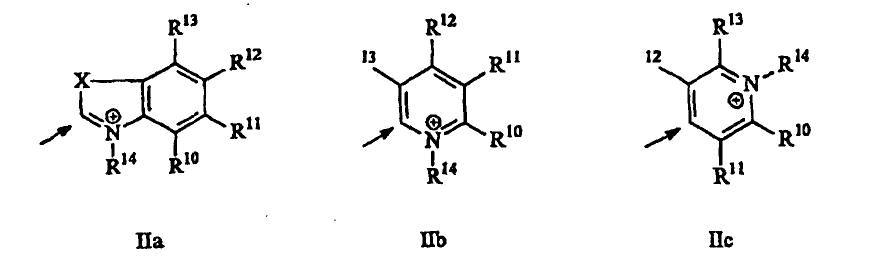 où
- X représente un élément du groupe O, S, Se ou l'élément structurel N-alkyle ou C(alkyle)₂,
- n représente la valeur numérique 0, 1, 2 ou 3,
- R¹-R¹⁴ sont identiques ou différents et peuvent de l'hydrogène, un ou plusieurs restes alkyle ou aryle, hétéroaryle ou hétérocycloaliphatique, un radical hydroxy ou alcoxy, une fonction amine substitué par un groupe alkyle ou cyclique et/ou deux restes en position ortho, par exemple R¹⁰ et R¹¹, peuvent former ensemble un autre cycle aromatique,
- au moins un des substituants R¹-R¹⁴ peut représenter un substituants solubilisant ou ionisable ou ionisé, comme cyclodextrine, sucre, SO₃-, PO₃²⁻, COO⁻, ou NR₃⁺, qui détermine les propriétés hydrophiles de ce colorant, où ce substituant peut également être lié au colorant marqueur par un groupe espaceur,
- au moins un des substituants R¹-R¹⁴ peut représenter un groupe réactif qui permet une liaison covalente du colorant avec une autre molécule, où ce substituant peut également être lié au colorant marqueur par un groupe espaceur, et
- R¹ représente un substituant qui présente en position α du cycle pyranne, un atome C quaternaire, où les substituants R¹ et R² peuvent former également un système cyclique aliphatique ou aliphatique substitué.

2. Colorants marqueurs NIR compatibles laser selon la revendication 1, **caractérisés en ce que** le groupe réactif est choisi parmi les fonctionnalités suivantes : isothiocyanate, isocyanate, monochlorotriazine, dichlorotriazine, aziridine, halogénure de sulfonyle, chlorure d'acide carboxylique, ester N-hydroxysuccinimide, imidoester, glyoxal ou aldéhyde de fonctions amine et hydroxy ou maléimide ou iodoacétamide pour des fonctions thiol ainsi que phosphoramidite pour le marquage de l'ADN ou de l'ARN ou de leurs fragments.

3. Colorants marqueurs NIR compatibles laser selon la revendication 1 ou 2, **caractérisés en ce que** le groupe réactif est lié par un groupe espaceur de la formule générale -(CH₂)ₘ- sur le chromophore proprement dit, où la valeur de m peut être de 1 à 18.

4. Colorants marqueurs NIR, compatibles laser selon la revendication 1 à 3, **caractérisés en ce que** l'unité structurelle =CR⁸- contient également un pontage par des systèmes cycliques à quatre, cinq et six chaînons, où se trouvent également sur ceux-ci des groupes réactifs et les substituants A - G peuvent présenter la même fonctionnalité que les substituants R¹-R¹⁴.

5. Colorants marqueurs NIR compatibles laser selon la revendication 4, **caractérisés en ce que** l'unité structurelle =CR⁸- représente :

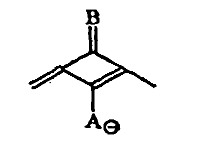

6. Colorants marqueurs NIR compatibles laser selon la revendication 4, **caractérisés en ce que** l'unité structurelle =CR⁸- représente :

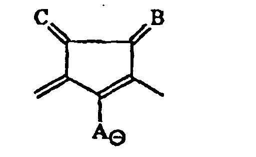

7. Colorants marqueurs NIR compatibles laser selon la revendication 4, **caractérisés en ce que** l'unité structurelle =CR⁸- représente :

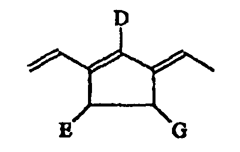

8. Colorants marqueurs NIR compatibles laser selon la revendication 4, **caractérisés en ce que** l'unité structurelle =CR⁸- représente :

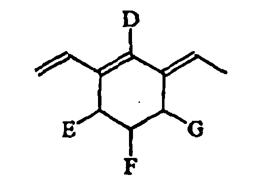

9. Colorants marqueurs NIR compatibles laser selon la revendication 1-8, **caractérisés en ce que** les substituants A - G peuvent présenter la même structure que les substituants R¹-R¹⁴, ou peuvent représenter O, S, C(CN)₂ ou N-R, où R dans N-R peut représenter un reste aliphatique ou aromatique, ou un reste aliphatique ou aromatique réactif, comme (CH₂)ₙCOOH ou (CH₂)ₙNH₂.

10. Colorants marqueurs NIR compatibles laser selon la revendication 1-8, **caractérisés en ce que** le substituant D représente Cl, un reste alkyle, alcoxy, cycloalcoxy, phénolate, alkylmercapto ou phénylmercapto, sur lequel des substituants le cas échéant réactifs correspondant aux substituants R¹ à R¹⁴ sont introduits.

11. Colorants marqueurs NIR, compatibles laser selon la revendication 1, **caractérisés en ce que** le substituant R¹ représente un groupe 1,1-diméthyléthyle, 6,6-diméthylbicyclo[3.1.1]hept-2-èn-2-yle, - bicyclo[2.1.1]hept-2-èn-5-yle, 3,3-diméthylbut-1-èn-1-yle ou adamant-1-yle (-C₁₀H₁₅/tricyclo[3.3.1.1^{3,7}]décyle).

12. Colorants marqueurs NIR compatibles laser selon la revendication 1, **caractérisés en ce que** le substituant R⁵ représente une amine une ou deux fois alkylée, où par l'azote d'amine, des pontages alkyle et alcényle avec les substituants R⁴ et R⁶ sont possibles.

13. Colorants marqueurs NIR compatibles laser selon les revendications 1-12, **caractérisés en ce que** la partie ω-(benzo[b]pyrann-4-ylidène)alc-1-ényle du colorant possède la structure Ia, Ib, Ic ou Id suivante : 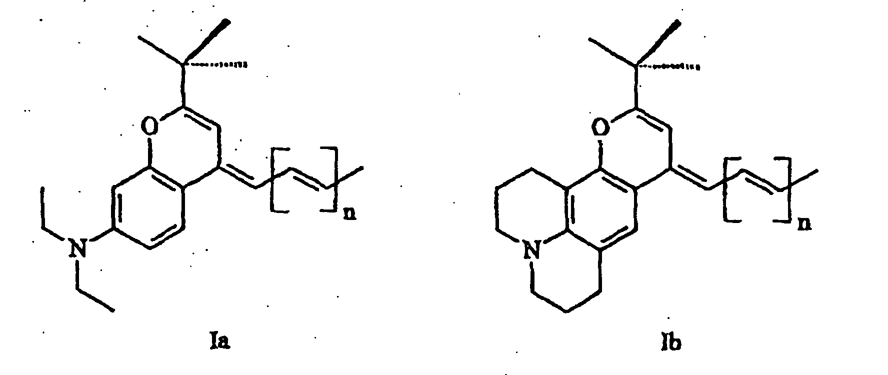 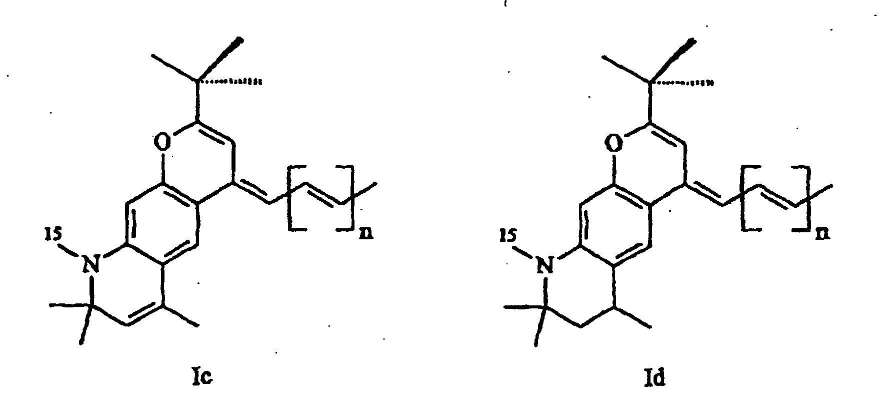 où le substituant R¹⁵ peut posséder les mêmes fonctionnalités que les substituants R¹ à R¹⁴.

14. Colorant marqueur compatible laser selon les revendications 1-13, de sorte que
a. R², R³, R⁴, R⁶, R⁷, R⁸, R⁹ sont H, R¹ est 1,1-diméthyléthyle, R⁵ est N,N-diéthylamino, n=1, Z est IIa avec R¹⁰, R¹¹, R¹³ qui sont H, R¹² est SO₃- et R¹⁴ est 5-carboxypent-1-yle, à savoir le 5-sulfonate de 1-(5-carboxypent-1-yl)-3,3-diméthyl-2-[3-(7-N,N-diéthylamino-2-(1,1-diméthyléthyl)-4H-benzopyrann-4-ylidène)-1-propène-1-yl]-3H-indolium (OB11),
b. R², R³, R⁷, R⁸, R⁹ sont H, R⁴, R⁵, R⁶ forment ensemble, un cycle julolidine, R¹ est 1,1-diméthyléthyle, n=1, Z est IIa avec R¹⁰, R¹¹, R¹³ qui sont H, R¹² est SO₃- et R¹⁴ est 5-carboxypent-1-yle, à savoir le 5-sulfonate de 1-(5-carboxypentyl)-3,3-diméthyl-2-[3-(11-(2,2-diméthyléthyl)-1H,2H,3H,5H,6H,7H-pyranno[2,3-f]pyrido[3,2,1-ij]quinoléin-9-ylidène)-1-propényl]-3H-indolium (OB15),
c. R², R³, R⁴, R⁶, R⁷, R⁸, R⁹ sont H, R¹ est 1,1-diméthyléthyle, R⁵ est N,N-diéthylamino, n=1, Z est IIc avec R¹⁰, R¹¹ qui sont H, R¹² et R¹³ qui forment ensemble un cycle condensé de structure CH=C(SO₃⁻)-CH=CH et R¹⁴ est 5-carboxypent-1-yle, à savoir le 6-sulfonate de 1-(5-carboxypent-1-yl)-4-[3-(7-N,N-diéthylamino-2-(1,1-diméthyléthyl)-4H-benzopyrann-4-ylidène)-1-propène-1-yl]quinoléinium (OB20),
d. R², R³, R⁴, R⁶, R⁷, R⁸, R⁹ sont H, R¹ est 1,1-diméthyléthyle, R⁵ est N,N-diéthylamino, n=1, Z est IIc avec R¹⁰, R¹¹ qui sont H, R¹² et R¹³ qui forment ensemble un cycle condensé de structure CH=CH-CH=CH et R¹⁴ est 3-hydroxyprop-1-yle, à savoir le perchlorate de 1-(3-hydroxyprop-1-yl)-4-[3-(7-N,N-diéthylamino-2-(1,1-diméthyléthyl)-4H-benzopyrann-4-ylidène)-1-propène-1-yl]quinoléinium (OB 14).

15. Utilisation de dérivés substitués du pyranne de formule générale I selon la revendication 1, comme colorants pour le marquage optique de protéines, d'acides nucléiques, d'oligomères, d'ADN, d'ARN, de cellules biologiques, de lipides, de polymères, de composés pharmaceutiques ou de particules polymères.

16. Système pour la détermination qualitative ou quantitative de protéines, d'acides nucléiques, d'oligomères, d'ADN, d'ARN, de cellules biologiques, de lipides, de polymères, de composés pharmaceutiques ou de particules polymères, **caractérisé en ce que** les groupes fonctionnels des composés selon les revendications 1 à 14 sont couplés de manière covalente sur une fonction OH, NH₂ ou SH des substances à déterminer.

17. Système selon la revendication 16, **caractérisé en ce que** la réaction de couplage est réalisée en solution aqueuse.

18. Système selon la revendication 16 ou 17, **caractérisé en ce que** le composé lié de manière covalente présente des propriétés fluorescentes.

19. Utilisation des composés et systèmes selon les revendications 1 à 18, dans des procédés qualitatifs et quantitatifs, de détermination optiques, en particulier fluorescents, y compris des tests immunologiques, des procédés d'hybridation, des procédés chromatographiques ou électrophorétiques et des criblages à haut débit.

20. Utilisation des composés et systèmes selon les revendications 1 à 18, pour l'analyse d'interactions récepteur-ligand sur un microassemblage.
